# EUROPEAN PATENT APPLICATION

(11) **EP 4 388 973 A1**
(43) Date of publication of application: **26.06.2024**
(21) Application number: 22216505.2
(22) Date of filing: 23.12.2022
(51) Int. Cl.: A61B 1/00, A61B 1/04, G02B 23/24, H04N 7/18

(54) **APPARATUS FOR AN OPTICAL IMAGING SYSTEM, DEVICE FOR A DISPLAY DEVICE, OPTICAL IMAGING SYSTEM, METHOD AND COMPUTER PROGRAM**

(71) Applicant: Leica Instruments (Singapore) Pte Ltd, Singapore 608924 (SG)
(72) Inventor: ROSTYKUS, Manon, 1085 Vulliens (CH)
(74) Representative: 2SPL Patentanwälte PartG mbB

(57) **Abstract**

Examples relate to an apparatus 130 for an optical imaging system. The apparatus 130 comprises one or more processors 134 and one or more storage devices 136. The apparatus 130 is configured to receive information about a selection of a display device 300 for displaying an image of the optical imaging system. Further, the apparatus is configured to generate a control signal for controlling a radiation of an indication element 310 of the selected display device 300 to indicate the selection and to transmit the control signal to the indication element 310.

## Description

### Technical field

Examples relate to an apparatus for an optical imaging system, a device for a display device, an optical imaging system, a method and a computer program.

### Background

In an optical imaging system, e.g., a surgical imaging system, a surgeon often uses a main pair of oculars or head-mounted displays to perform a surgical procedure. In many cases, the surgeon does not perform the surgical procedure alone, but with help of an assistant. For this purpose, the optical imaging system may feature further pairs of oculars or head-mounted displays. The view through the head-mounted displays may be different for each user. Thus, there may be a desire for an improved concept to maintain multiple pairs of oculars or head-mounted displays of an optical imaging system.

### Summary

This desire is addressed by the subject-matter of the independent claims.

The concept proposed in the present disclosure is based on the insight that maintaining multiple head-mounted displays can be improved by generating a control signal to control a radiation of an indication element. The indication element can indicate several parameters, such as a selection of the head-mounted device. In this way, a user, e.g., an assistant, can be informed about a selected head-mounted device.

Examples provide an apparatus for an optical imaging system. The apparatus comprises one or more processors and one or more storage devices. The apparatus is configured to receive information about the selection of the display device. The display device is for displaying an image of the optical imaging system. For example, the image may be a view of a sample under examination, e.g., being observed through a microscope of the optical imaging system. The apparatus is further configured to generate a control signal for controlling a radiation of an indication element of the selected display device to indicate the selection. Further, the apparatus is configured to transmit the control signal to the indication element. By generating a control signal the apparatus can control the radiation of the indication element. Thus, the apparatus can change the radiation of the indication element to indicate the selection of the display device, for example. In this way, a user can be informed about the selection of the display device in a simplified way.

In an example, the apparatus may be further configured to obtain status information about the status of the selected display device. For example, the status of the display device may be an operating status. Further, the apparatus may be configured to generate a status signal for controlling the radiation of the indication element of the selected display device to indicate the status of the selected display device. The generation of the status signal may be based on the status information. The apparatus may be further configured to transmit the status signal to the indication element. In this way, a user, e.g., an assistant, can be informed about a status of the selected display device in a simplified way. The status signal may be a different signal as the control signal. Alternatively, the status signal may be part of the control signal.

In an example, the control signal may be for changing the radiation of the indication element. For example, a radiation of the indication element may be activated and/or changed to indicate the selection and/or the status of the display device. In this way, a user can be informed about different parameters of the display device in a simplified way.

In an example, the status signal may be indicative of an error in receiving information about the image. The information may be received from the optical imaging system, e.g., from a sensor of the optical imaging system. In this way, a user can be informed about an error in the signal path and/or of image acquisition of the sample. Thus, the user can take certain measures to counteract the error.

In an example, the status signal may be indicative of an erroneous status of the selected display device. In this way, the user can be informed about an error of the display device. Thus, the user can take certain measures to counteract the error.

In an example, the indication element may comprise at least one of a loudspeaker or a light source. In this way, the information about the selection of the display device can be provided to the user in an appropriate way.

In an example, the indication element may comprise the light source. Further, the control signal may be indicative of at least one of a wavelength, a blink rate or an intensity of light to be radiated by the indication element. In this way, the information about the selection and/or the status of the display device can be provided to the user in an appropriate way.

In an example, the apparatus may be configured to receive the information about the selection from a user input. For example, the user input can be provided on a monitor. In this way, the user can use the monitor to select the display device and optionally can adjust a setting of the display device on the monitor.

Examples provide a device for a display device. The device comprises one or more processors and one or more storage devices. The device is configured to receive a control signal for controlling a radiation of an indication element of the display device to indicate a selection of the display device. The control signal is received from an apparatus as described above. Further, the device is configured to control the radiation of the indication element of the display device based on the control signal. The indication element can be controlled by the device to indicate a selection of the display device. In this way, the user can be informed about the selection of the display device in a simplified way.

Examples provide a display device comprising an apparatus as described above and/or a device as described above and an indication element. Thus, the display device can be configured to indicate a selection of its own.

Examples provide an optical imaging system comprising an apparatus as described above, a device as described above and/or a display device as described above.

Examples provide a method for an apparatus for an optical imaging system for indicating a selection of a display device communicatively coupled to the optical imaging system. The method comprises receiving information about a selection of a display device for displaying an image of the optical imaging system. Further, the method comprises generating a control signal for controlling a radiation of an indication element of the selected display device to indicate the selection. The method further comprises transmitting the control signal to the indication element.

Examples provide a method for a device for indicating a selection of a display device communicatively coupled to an optical imaging system. The method comprises receiving a control signal for controlling an indication element of the display device to indicate a selection of the display device. The control signal is received from an apparatus as described above. Further, the method comprises controlling the radiation of the indication element based on the control signal.

Various examples of the present disclosure relate to a corresponding computer program with a program code for performing the above method when the computer program is executed on a processor.

### Short description of the Figures

Some examples of apparatuses and/or methods will be described in the following by way of example only, and with reference to the accompanying figures, in which
- Figs. 1a and 1b: show schematic diagrams of an example of an apparatus for an optical imaging system and of a corresponding optical imaging system comprising the apparatus;
- Fig. 2: shows a block diagram of an example of a device;
- Figs. 3a and 3b: show an example of a display device and a selection of the display device;
- Fig. 4: shows an example of a method for an apparatus for an optical imaging system for indicating a selection of a display device communicatively coupled to the optical imaging system;
- Fig. 5: shows an example of a method for a device for indicating a selection of a display device communicatively coupled to an optical imaging system; and
- Fig. 6: shows a schematic diagram of a system comprising a microscope and a computer system.

### Detailed Description

Various examples will now be described more fully with reference to the accompanying drawings in which some examples are illustrated. In the figures, the thicknesses of lines, layers and/or regions may be exaggerated for clarity.

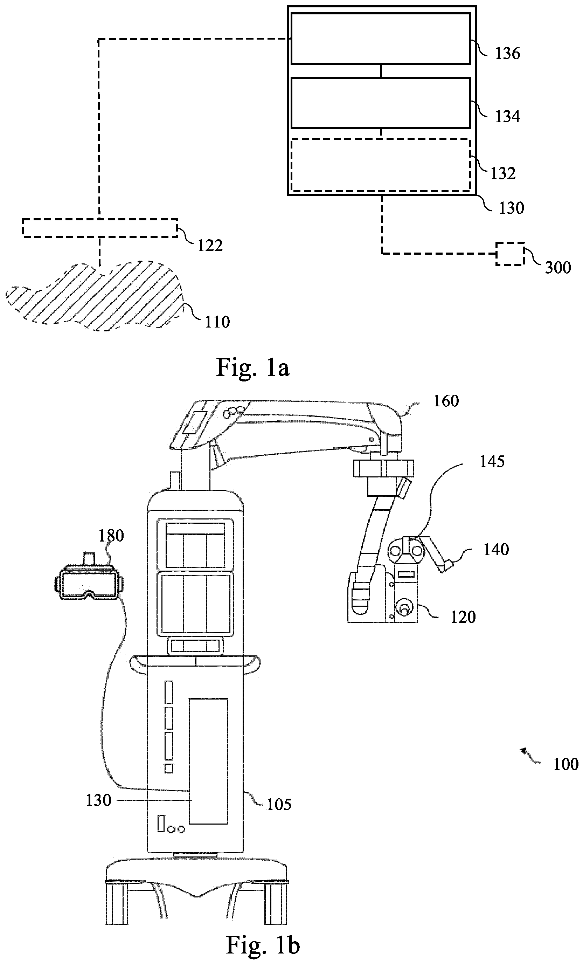

Figs. 1a and 1b show schematic diagrams of an example of an apparatus 130 for an optical imaging system 100 and of a corresponding optical imaging system 100 comprising the apparatus 130. The apparatus 130 is tasked with controlling various aspects of a microscope 120 of the optical imaging system 100, which may be a surgical imaging system, and of the entire optical imaging system 100 and/or with processing various types of sensor data of the optical imaging system 100. Consequently, the apparatus 130 may be implemented as a computer system, which interfaces with the various components of the optical imaging system, e.g., the sensor 122.

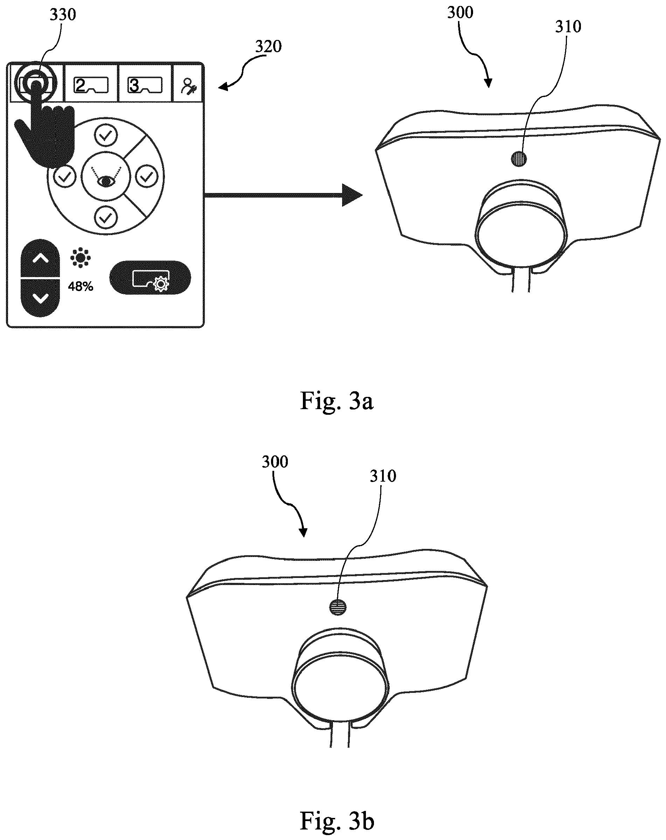

The apparatus 130 comprises, as shown in Fig. 1a, one or more processors 134 and one or more storage devices 136. Optionally, the apparatus 130 further comprises one or more interfaces 132. The one or more processors 134 are coupled to the one or more storage devices 136 and to the optional one or more interfaces 132. In general, the functionality of the apparatus 130 may be provided by the one or more processors 134 (for generating the control signal), in conjunction with the one or more interfaces 132 (for exchanging information, e.g., with the sensor 122 or an indication element as shown in Fig. 3, e.g., to transmit the control signal to the indication element) and/or with the one or more storage devices 136 (for storing and/or retrieving information).

The apparatus 130 is configured to receive information about the selection of the display device 300. The selection of the display device 300 is for displaying an image of the optical imaging system, e.g., an image generated by the one or more processors 134, using the display device 300. For example, the information about the selection may be received from an input device, such like a touch display. A user may use the touch display to maintain a setting of the display device 300. In this way, selecting of the display device 300 can be facilitated, since the user may use the touch display to select the display device 300.

Additionally or alternatively, the information about the selection may be received from the display device 300. For example, the display device 300 may comprise a motion sensor. The motion sensor may be configured to detect the motion of the display device 300. If a motion of the display device 300, e.g., a motion exceeding a threshold, is detected by the motion sensor, the display device 300 may transmit the information of the motion sensor to the apparatus 130. The apparatus 130 may determine the selection of the display device 300 based on the information of the motion sensor. In this way, the apparatus 130 can generate a control signal to indicate a status of the moved display device 300, for example.

Further, the apparatus 130 is configured to generate a control signal for controlling a radiation of indication element. The indication element is part of the selected display device 300. The indication element can be used to indicate the selection of the display device 300. The apparatus 130 is further configured to transmit the control signal to the indication element. The indication element can be used to indicate a working status of the selected display device 300 and/or to indicate the selected display device 300 if multiple display devices 300 are used, for example. In this way, a user of the optical imaging system 100 can be informed about (at least one) parameter of the selected display device 300 and/or about the selection of the display device 300 in a simplified way. In this way, the user can be enabled to control the correct display device 300, e.g., change settings of a desired display device.

The proposed concept is built around two main components - the microscope 120, which comprises the optical components, and which may house the display device 300 being used to view the sample 110, and the apparatus 130, which is used to control the optical imaging system 100, process sensor data of the microscope 120, e.g., the sensor 122, and to generate a control signal.

In general, a microscope, such as the microscope 120, is an optical instrument that is suitable for examining objects that are too small to be examined by the human eye (alone). For example, a microscope 120 may provide an optical magnification of a sample, such as a sample 110 shown in Fig. 1a. In modern microscopes, the optical magnification is often provided for a camera or an imaging sensor, such as the optical imaging sensors 122 of the microscope 120. The microscope 120 may further comprise one or more optical magnification components that are used to magnify a view of the sample 110, such as an objective.

There are a variety of different types of optical imaging systems. For example, a microscope, an exoscope, an endoscope may be an imaging device of an optical imaging system. If the optical imaging system 100 is used in the medical or biological fields, the sample 110 may be a sample of organic tissue, e.g., arranged within a petri dish or present in a part of a body of a patient. In some examples of the present disclosure, e.g., as shown in Fig. 1b, the optical imaging system 100 may be a surgical imaging system, e.g., a microscope system that is to be used during a surgical procedure, such as an oncological surgical procedure or during tumor surgery. However, the proposed concept may also be applied to other types of microscopy, e.g., microscopy in a laboratory or microscopy for the purpose of material inspection.

As is evident, the optical imaging system 100 comprises a number of components, such as the apparatus 130, the microscope 120 with the at least one optical imaging sensors 122, an optional main pair of ocular displays 140, an optional secondary pair of ocular displays 145 and a head-mounted display 180. In the configuration shown in Fig. 1a, the microscope 120 may be a non-stereoscopic microscope 120 that only supports a two-dimensional view of the sample 110 using the sensor 122. However, other implementations may be possible, such as an implementation with a single pair of optical imaging sensors for visible light imaging, or a pair of optical imaging sensors for visible light and fluorescence imaging, or four image sensors for both visible light (reflectance) imaging and fluorescence imaging, two optical imaging sensors for providing the two stereo channels for visible light imaging and two optical imaging sensors for providing the two stereo channels for fluorescence imaging.

Fig. 1b shows a schematic diagram of an example of a (surgical) optical imaging system 100 comprising the microscope 120 and the apparatus 130. In general, a (surgical) optical imaging system is a system that comprises a microscope 120 and additional components, which are operated together with the microscope 120. In other words, a (surgical) optical imaging system is a system that comprises the microscope 120 and one or more additional components, such as the apparatus 130 (which may be a computer system being adapted to control and, for example, generate the control signal), an illumination system (which is used to illuminate a sample being imaged by the microscope 120), additional sensors, displays etc.

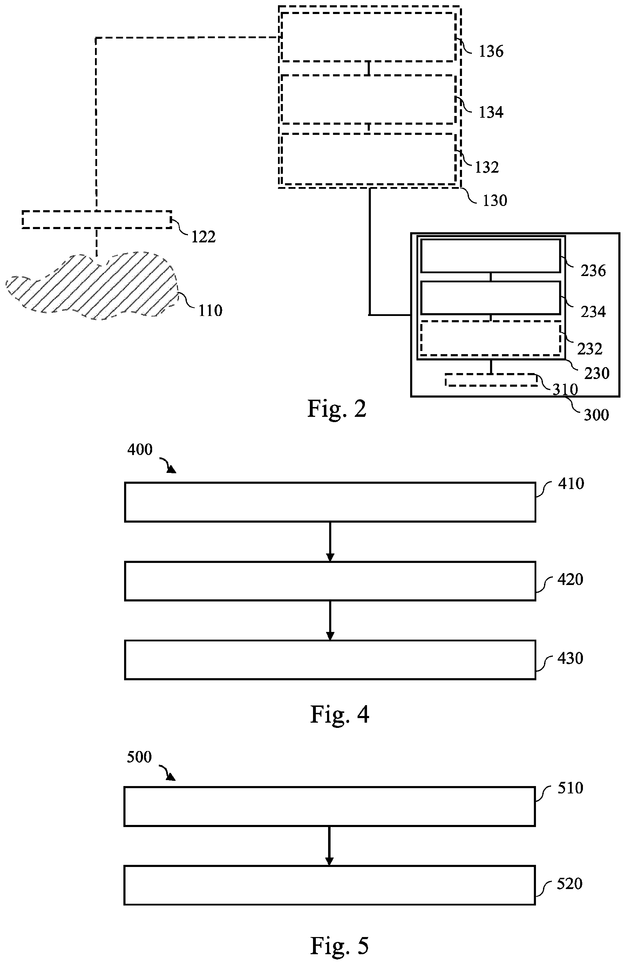

The optical imaging system 100 comprises an apparatus 130, a device as described in connection with Fig. 2 and/or a display device 300 as described in connection with Fig. 3.

The (surgical) optical imaging system 100 shown in Fig. 1b comprises a number of optional components, such as a base unit 105 (which may comprise the apparatus 130) with a stand, ocular displays 140; 145 that are arranged at the microscope 120, a head-mounted display 180 (which may comprise the apparatus 130), and a (robotic or manual) arm 160 which holds the microscope 120 in place, and which is coupled to the base unit 105 and to the microscope 120. In general, these optional and non-optional components may be coupled to the apparatus 130, which may be configured to control and/or interact with the respective components.

In Fig. 1b, the (surgical) optical imaging system 100 is shown with three display devices 300 - a primary pair of digital (or optical) oculars 140, which are used by the main surgeon, a secondary pair of digital oculars 145, which are to be used by the assistant that is positioned to the side of the microscope, and a head-mounted display 180, which may be virtual-, augmented- or mixed-reality reality goggles, which may also be used by the main surgeon or by the assistant (or another assistant). For example, the head-mounted display 180 may be a display device 300 that is to be worn by a person, e.g., the main surgeon or an assistant, on or around the head, such that one or two displays are arranged in front of the eye or eyes of the person. The head-mounted display 180 may be identical to the display device 300, e.g., to a display device 300 as shown in Fig. 3.

In an example, the apparatus 130 may be further configured to obtain status information about the status of the selected display device 300. Further, the apparatus 130 may be configured to generate a status signal for controlling the radiation of the indication element of the selected display device 300 to indicate the status of the selected display device 300. The generation of the status signal may be based on the status information. The apparatus may be further configured to transmit the status signal to the indication element.

The status of the display device 300 may be an operating status or working status of the display device 300. The indication element can be used to indicate different working stati such like a usable (the display device 300 may work properly) or an unusable working status (if there is a problem with the display device 300). For example, as described below the indication element may be a light emitting diode, LED. The LED can radiate a first wavelength, e.g., red light, to indicate an unusable working status and a second wavelength, e.g., green light, to indicate a usable working status, for example. Optionally or alternatively, the selection of the display device 300 can be indicated by a blink rate of the indication element. For example, the indication element of a selected display device 300 that is in a usable working status may blink with a green light. Thus, the user can be informed about the selection of the display device 300 and optionally about the status of the selected display device 300 in a simplified way.

In an example, the control signal and/or the status signal may be for changing the radiation of the indication element. The radiation of the indication element is the emission of energy in the form of waves or particles through space or through material medium. For example, the radiation may be an electromagnetic radiation, such like visible light and/or an acoustic radiation, such like sound. The radiation of the indication element can be activated to indicate the selected display device 300, e.g., a LED may be turned on. Alternatively, the radiation of the indication element can be changed, e.g., from red light to green light. Thus, the user can be informed in simplified way about the selection of the display device 300 and optionally about the status of the display device 300.

In an example, the status signal may be indicative of an error in receiving information about the image. The information may be received from the optical imaging system 100, e.g., from a sensor 122 of the optical imaging system 100. The information about the image may be raw data received from the optical imaging system 100 (e.g., raw data of the sensor 122). For example, the one or more processors 132 may generate the image based on the raw data received from the optical imaging system 100. If the one or more processors 132 may be unable to generate the image (e.g., because the raw data is corrupt or could not be fully received) the apparatus 130 can generate the status signal such that it is indicative of the error in the image generation. Thus, the indication element can be controlled to radiate to inform a user about the error in the image generation. In this way, the user can take certain measures to counteract the error in the image generation.

Alternatively, the apparatus 130 may receive the image from the optical imaging system 100 (e.g., the optical imaging system 100 may have generated the image based on sensor data of the sensor 122). If the apparatus 130 may be unable to receive the image from the optical imaging system 100, the apparatus 130 can generate a status signal such that it is indicative of the error in receiving the image.

In an example, the status signal may be indicative of an erroneous status of the selected display device 300. For example, the display device 300 can be in an unusable working status. If the user selects the unusable display device 300, the indication element can be used to inform the user about the unusable working status. Thus, the user can take certain measures to restore a usable working status. Alternatively, the user can select another display device 300. In this way, user experience can be increased. For example, if a user lifts a first unusable display device, the indication element may indicate the unusable status. This allows the user to lift another display device for use.

In an example, the indication element may comprise at least one of a loudspeaker or a light source. The loudspeaker can be used to radiate a sound. The light source, e.g., a LED, a Laser, can be used to radiate an electromagnetic wave, e.g., visible light.

In an example, the indication element may comprise or may be the light source. Further, the control signal may be indicative of at least one of a wavelength, a blink rate or an intensity of light to be radiated by the indication element. Thus, the user of the optical imaging system 100 can be informed in simplified way about the selection of the display device 300 and optionally about the status of the selected display device 300.

In an example, the apparatus may be configured to receive the information about the selection from a user input, see Fig. 3. For example, the user input can be provided on a computer, e.g., a touch display of a computer. In this way, the user can use the computer to select the display device 300 and optionally can adjust a setting of the display device 300 using the computer. The user may be an assistant, e.g., a service person, responsible for maintaining multiple display devices 300. For example, the service person may assist multiple surgeons during an operation. The service person may want to adjust a view of the image for a first display device 300. Thus, the service person can select on the computer a display device 300 and can be informed by the selected display device 300 about the selection. In this way, the service person can receive information whether the selection on the computer matches the desired display device 300.

As shown in Fig. 1a the optional one or more interfaces 132 are coupled to the respective one or more processors 134 at the apparatus 130. In examples the one or more processors 134 may be implemented using one or more processing units, one or more processing devices, any means for processing, such as a processor, a computer or a programmable hardware component being operable with accordingly adapted software. Similar, the described functions of the one or more processors 134 may as well be implemented in software, which is then executed on one or more programmable hardware components. Such hardware components may comprise a general-purpose processor, a Digital Signal Processor (DSP), a micro-controller, etc. The one or more processors 134 is capable of controlling the one or more interfaces 132, so that any data transfer that occurs over the one or more interfaces 132 and/or any interaction in which the one or more interfaces 132 may be involved may be controlled by the one or more processors 134.

In an embodiment the apparatus 130 may comprise a memory, e.g., the one or more storage devices 136 and at least one or more processors 134 operably coupled to the memory and configured to perform the method described below.

In examples the one or more interfaces 132 may correspond to any means for obtaining, receiving, transmitting or providing analog or digital signals or information, e.g., any connector, contact, pin, register, input port, output port, conductor, lane, etc. which allows providing or obtaining a signal or information. The one or more interfaces 132 may be wireless or wireline and it may be configured to communicate, e.g., transmit or receive signals, information with further internal or external components.

The apparatus 130 may be a computer, processor, control unit, (field) programmable logic array ((F)PLA), (field) programmable gate array ((F)PGA), graphics processor unit (GPU), application-specific integrated circuit (ASICs), integrated circuits (IC) or system-on-a-chip (SoCs) system. The apparatus 130 may be part of the display device 300. Alternatively, the apparatus 130 may be extern to the display device 300 and may communicate with a device as described with reference to Fig. 2.

More details and aspects are mentioned in connection with the examples described below. The example shown in Fig. 1 may comprise one or more optional additional features corresponding to one or more aspects mentioned in connection with the proposed concept or one or more examples described below (e.g., Fig. 2 - 6).

Fig. 2 shows a block diagram of an example of a device 230. The device 230 for a display device 300 may be a counterpart of the apparatus as described in Fig. 1. For example, the device 230 may be a controller of the display device 300, e.g., to control an indication element of the display device. The device 230 comprises one or more processors 234 and one or more storage devices 236. The device 230 is configured to receive a control signal for controlling a radiation of an indication element 310 of the display device 300 to indicate a selection of the display device 300. The control signal is received from an apparatus as described in Fig. 1. Further, the device 230 is configured to control the radiation of the indication element 310 of the display device 300 based on the control signal. In this way, the device 230 can control the indication element 310 of the display device 300. Computation resource intensive task, such like generate the control signal or optionally generate the image of the optical imaging system can be performed by the apparatus 130. Further, a data transfer between the optical imaging system and the display device 300 can be reduced.

As shown in Fig. 2 the optional one or more interfaces 232 are coupled to the respective one or more processors 234 at the device 230. In examples the one or more processors 234 may be implemented using one or more processing units, one or more processing devices, any means for processing, such as a processor, a computer or a programmable hardware component being operable with accordingly adapted software. Similar, the described functions of the one or more processors 234 may as well be implemented in software, which is then executed on one or more programmable hardware components. Such hardware components may comprise a general-purpose processor, a Digital Signal Processor (DSP), a micro-controller, etc. The one or more processors 234 is capable of controlling the one or more interfaces 232, so that any data transfer that occurs over the one or more interfaces 232 and/or any interaction in which the one or more interfaces 232 may be involved may be controlled by the one or more processors 234.

In an embodiment the device 230 may comprise a memory, e.g., the one or more storage devices 236 and at least one or more processors 234 operably coupled to the memory and configured to perform the method described below.

In examples the one or more interfaces 232 may correspond to any means for obtaining, receiving, transmitting or providing analog or digital signals or information, e.g., any connector, contact, pin, register, input port, output port, conductor, lane, etc. which allows providing or obtaining a signal or information. The one or more interfaces 232 may be wireless or wireline and it may be configured to communicate, e.g., transmit or receive signals, information with further internal or external components.

The device 230 may be a computer, processor, control unit, (field) programmable logic array ((F)PLA), (field) programmable gate array ((F)PGA), graphics processor unit (GPU), application-specific integrated circuit (ASICs), integrated circuits (IC) or system-on-a-chip (SoCs) system.

More details and aspects are mentioned in connection with the examples described above and/or below. The example shown in Fig. 2 may comprise one or more optional additional features corresponding to one or more aspects mentioned in connection with the proposed concept or one or more examples described above (e.g., Fig. 1) and/or below (e.g., Fig. 3 - 6).

Figs. 3a and 3b show an example of a display device 300 and a selection of the display device 300.

As can be seen in Fig. 3a an input device, e.g., a monitor of a laptop such as a touch screen, a keyboard, a touch pad, can be used to receive an input of a user of the display device 300. The user may provide an input using a graphic user interface, GUI, 320. For example, the GUI 320 may comprise different symbols for different display devices 300. The user can select the tab 330 associated with the first display device 300. The information about selection of the display device 300 may be transmitted to the apparatus as described in Fig. 1. The apparatus generates a control signal and transmits the control signal to the indication element 310 of the display device 300. The indication element 310 can be a LED. The LED may blink with green light to indicate the selection.

For example, the GUI 320 may run on the controller (also referred to as hub). Several display devices may be linked to the hub the GUI 320. The hub may provide a user an improved functionality for the display devices, e.g., to adjust the setting of the display device. Due to the GUI 320 running on the hub, when a user of the hub is pressing the tab 330 corresponding to the display device 300, the indication element 310 of the display device 300 may blink green (indicated by the vertically hatching) to inform the user which display device is associated with the tab 330. In this way, the user can easily select a desired display device.

As can be seen in Fig. 3b the display device 300 comprises an indication element 310. Further, the display device 300 comprises an apparatus as described in Fig. 1 and/or a device as described in Fig. 2 (not shown). Further, the indication element 310 may radiate red light (indicated horizontally hatching). The radiation of red light may be indicative of an unusable working status. Alternatively, the indication element 310 may radiate green light, which may be indicative of usable working status. For example if the display device 300 is selected the indication element 310 may either blink red (unusable) or green (usable). In this way, the user can be informed about selection and a working status of the display device in a simplified way.

More details and aspects are mentioned in connection with the examples described above and/or below. The example shown in Fig. 3 may comprise one or more optional additional features corresponding to one or more aspects mentioned in connection with the proposed concept or one or more examples described above (e.g., Fig. 1-2) and/or below (e.g., Fig. 4 - 6).

Fig. 4 shows an example of a method 400 for an apparatus for an optical imaging system for indicating a selection of a display device communicatively coupled to the optical imaging system. The method 400 comprises receiving 410 information about a selection of a display device for displaying an image of the optical imaging system. Further, the method 400 comprises generating 420 a control signal for controlling a radiation of an indication element of the selected display device to indicate the selection. The method 400 further comprises transmitting 430 the control signal to the indication element. The method 400 may be performed by an apparatus as described in Fig. 1. Accordingly, features introduced in connection with the apparatus shown in Fig. 1 may likewise be included in the method of Fig. 4.

More details and aspects are mentioned in connection with the examples described above and/or below. The example shown in Fig. 4 may comprise one or more optional additional features corresponding to one or more aspects mentioned in connection with the proposed concept or one or more examples described above (e.g., Fig. 1-3) and/or below (e.g., Fig. 5 - 6).

Fig. 5 shows an example of a method 500 for a device for indicating a selection of a display device communicatively coupled to an optical imaging system. The method 500 comprises receiving 510 a control signal for controlling an indication element of the display device to indicate a selection of the display device. The control signal is received from an apparatus as described in Fig. 1. Further, the method 500 comprises controlling 520 the radiation of the indication element based on the control signal. The method 500 may be performed by the device as described in Fig. 2. Accordingly, features introduced in connection with the device shown in Fig. 2 may likewise be included in the method of Fig. 5.

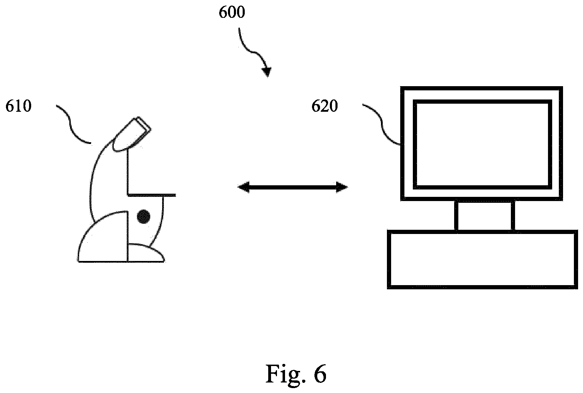

More details and aspects are mentioned in connection with the examples described above and/or below. The example shown in Fig. 5 may comprise one or more optional additional features corresponding to one or more aspects mentioned in connection with the proposed concept or one or more examples described above (e.g., Fig. 1 - 4) and/or below (e.g., Fig. 6).

Some embodiments relate to a microscope comprising an apparatus as described in connection with one or more of the Figs. 1 to 5. Alternatively, a microscope may be part of or connected to an apparatus as described in connection with one or more of the Figs. 1 to 5. Fig. 6 shows a schematic illustration of a system 600 configured to perform a method described herein, e.g., with reference to Figs. 4 or 5. The system 600 comprises a microscope 610 and a computer system 620. The microscope may comprise the apparatus as described above, e.g., with reference to Fig. 1 and/or the device as described above, .e.g., with reference to Fig. 2. The microscope 610 is configured to take images and is connected to the computer system 620. The computer system 620 is configured to execute at least a part of a method described herein. The computer system 620 may be configured to execute a machine learning algorithm. The computer system 620 and microscope 610 may be separate entities but can also be integrated together in one common housing. The computer system 620 may be part of a central processing system of the microscope 610 and/or the computer system 620 may be part of a subcomponent of the microscope 610, such as a sensor, an actor, a camera or an illumination unit, etc. of the microscope 610.

The computer system 620 may be a local computer device (e.g., personal computer, laptop, tablet computer or mobile phone) with one or more processors and one or more storage devices or may be a distributed computer system (e.g., a cloud computing system with one or more processors and one or more storage devices distributed at various locations, for example, at a local client and/or one or more remote server farms and/or data centers). The computer system 620 may comprise any circuit or combination of circuits. In one embodiment, the computer system 620 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g., camera) or any other type of processor or processing circuit. Other types of circuits that may be included in the computer system 620 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 620 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 620 may also include a display device, one or more speakers, and a keyboard and/or controller, which can include a mouse, trackball, touch screen, voice-recognition device, or any other device that permits a system user to input information into and receive information from the computer system 620.

More details and aspects are mentioned in connection with the examples described above. The example shown in Fig. 6 may comprise one or more optional additional features corresponding to one or more aspects mentioned in connection with the proposed concept or one or more examples described above (e.g., Fig. 1 - 5).

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

The following claims are hereby incorporated in the detailed description, wherein each claim may stand on its own as a separate example. It should also be noted that although in the claims a dependent claim refers to a particular combination with one or more other claims, other examples may also include a combination of the dependent claim with the subject matter of any other dependent or independent claim. Such combinations are hereby explicitly proposed, unless it is stated in the individual case that a particular combination is not intended. Furthermore, features of a claim should also be included for any other independent claim, even if that claim is not directly defined as dependent on that other independent claim.

The aspects and features described in relation to a particular one of the previous examples may also be combined with one or more of the further examples to replace an identical or similar feature of that further example or to additionally introduce the features into the further example.

### List of reference Signs

100 optical imaging system
105 base
110 sample
120 microscope
122 sensor
130 apparatus
132 interface
134 processor
136 storage device
140, 145 ocular display
160 arm
180 head-mounted display
230 device
232 interface
234 processor
236 storage device
300 display device
310 indication element
320 GUI
330 tab
400 method for an optical imaging system for indicating a selection of a display device
410 receiving information about a selection of a display device
420 generating a control signal for controlling a radiation of an indication element
430 transmitting the control signal
500 method for a device for indicating a selection of a display device
510 receiving a control signal
520 controlling the radiation of the indication element
600 system
610 microscope
620 computer system

## Claims

1. An apparatus (130) for an optical imaging system, comprising one or more processors (134) and one or more storage devices (136), wherein the apparatus (130) is configured to:
receive information about a selection of a display device (300) for displaying an image of the optical imaging system;
generate a control signal for controlling a radiation of an indication element (310) of the selected display device (300) to indicate the selection; and
transmit the control signal to the indication element (310).

2. The apparatus (130) according to claim 1, further configured to:
obtain status information about a status of the selected display device (300);
generate, based on the status information, a status signal for controlling the radiation of the indication element (310) of the selected display device (300) to indicate the status of the selected display device (300); and
transmit the status signal to the indication element (310).

3. The apparatus (130) according to claim 2, wherein
the status signal is for changing the radiation of the indication element (310).

4. The apparatus (130) according to claim 2 or 3, wherein
the control signal is for changing the radiation of the indication element (310).

5. The apparatus (130) according to claim 2, 3 or 4, wherein
the status signal is indicative of an error in receiving, from the optical imaging system, information about the image.

6. The apparatus (130) according to claim 2, 3, 4 or 5, wherein
the status signal is indicative of an erroneous status of the selected display device (300).

7. The apparatus (130) according to any one of the preceding claims, wherein
the indication element (310) comprises at least one of a loudspeaker or a light source.

8. The apparatus (130) according to claim 7, wherein
the indication element (310) comprises the light source; and
the control signal is indicative of at least one of a wavelength, a blink rate or an intensity of light to be radiated by the indication element (310).

9. The apparatus (130) according to any one of the preceding claims, wherein
the apparatus (130) is configured to receive the information about the selection from a user input.

10. A device (230) for a display device (300), comprising one or more processors (252) and one or more storage devices (254), wherein the device (230) is configured to:
receive, from an apparatus (130) according to any one of the preceding claims, a control signal for controlling a radiation of an indication element (310) of the display device (300) to indicate a selection of the display device (300); and
control the radiation of the indication element (310) of the display device (300) based on the control signal.

11. A display device (300), comprising:
an apparatus (130) according to any one of the claims 1-9 or a device (150) according to the claim 10; and
an indication element (310).

12. An optical imaging system (100), comprising:
at least one of an apparatus (130) according to any one of the claims 1-9, a device (230) according to claim 10 or a display device (300) according to claim 11.

13. A method (400) for an apparatus for an optical imaging system for indicating a selection of a display device communicatively coupled to the optical imaging system, comprising:
receiving (410) information about a selection of a display device for displaying an image of the optical imaging system;
generating (420) a control signal for controlling a radiation of an indication element of the selected display device to indicate the selection; and
transmitting (430) the control signal to the indication element.

14. A method (500) for a device for indicating a selection of a display device communicatively coupled to an optical imaging system, comprising:
receiving (510), from an apparatus according to any one of the claims 1-8, a control signal for controlling an indication element of the display device to indicate a selection of the display device; and
controlling (520) the radiation of the indication element based on the control signal.

15. A computer program having a program code for performing a method according to claim 13 or 14 when the program is executed on processor.
